# EUROPEAN PATENT SPECIFICATION

(11) **EP 0 984 796 B1**
(45) Date of publication and mention of the grant of the patent: **27.07.2005**
(21) Application number: 97947218.0
(22) Date of filing: 05.12.1997
(51) Int. Cl.: A61L 27/00, A61F 2/28, A61C 8/00, A61C 13/00, A61C 13/08, A61L 27/34

(54) **IMPLANTABLE ARTIFICIAL TOOTH COATED WITH CHITOSAN**
IMPLANTIERBARER KÜNSTLICHER ZAHN BESCHICHTET MIT CHITOSAN
DENT ARTIFICIELLE IMPLANTABLE REVETUE DE CHITOSAN

(30) Priority: 26.04.1997 KR 9716481
(43) Date of publication of application: 15.03.2000
(73) Proprietor: Lee, Yong Chan, Kangnam-ku, Seoul 135-270 (KR)
(72) Inventor: Lee, Yong Chan, Kangnam-ku, Seoul 135-270 (KR)
(74) Representative: Evens, Paul Jonathan
(86) International application number: PCT/KR1997/000258
(87) International publication number: WO 1998/048861

(56) References cited:
- WO-A-91/18558
- WO-A-96/02259
- US-A- 4 547 327
- US-A- 5 344 654

## Description

### BACKGROUND OF THE INVENTION

### Field of the Invention

The present invention relates, in general, to a medical implant and, more particularly, to an implant coated with a polymeric material of hydrophilicity and osteoconductivity.

### Description of the Prior Art

In medicine, an implant is a device intended to be embedded in a tissue for therapeutic purpose or its desired aim. Generally, after an implant is embedded in a predetermined tissue site of a living body, e.g. nasal bone, dental root or other type bones, the integration of the implant is accomplished either through a medium of soft tissue between the implant and the tissue or by filling this site with a bone only without any intermediate. The latter case is called complete osseointegration.

After implantation in a bone tissue, the therapeutic purpose of the implant can be achieved with a higher possibility as the interface between the implant and its surrounding bone is filled with a larger amount of bony material. In conventional techniques, the osseointegration between an implant and a bone was found to be only about 60% and thus, there is a limit in the therapy with implant.

US 5 344 654 discloses a prosthetic device coated with an osteogenic protein to enhance the strength of the resultant bond between the prosthesis and the existing bone.

WO 91/18558 relates to synthetic implants which induce osteogenesis in vivo using an osteogenic protein dispersed within a porous matrix.

WO 96/02259 discloses the use of chitosan and a polysaccharide immobilized thereto as an agent capable of providing stimulated regeneration of hard tissue, for example in connection with implants in bone tissue.

US 4 547 327 relates to a porous implantable oral prosthesis according to the preamble of claim 1.

### SUMMARY OF THE INVENTION

It is an object of the present invention to overcome the above problems encountered in prior arts and to provide a medical implant which is greatly improved in osseointegration as well as in stability in the early stage of the implantation.

In accordance with the present invention, there is provided an artificial tooth as defined in appendent claim 1 to which reference should now be made.

### DETAILED DESCRIPTION OF THE INVENTION

Chitosan, a natural polymer which can be relatively easily obtained from the nature, shows both hydrophilicity and osteoconductivity. These properties enable chitosan to be used as a medical implant. When chitosan is placed in depth of a body, the polymeric compound absorbs moisture from the body, swelling up. This volumetric increase of the polymeric compound brings about an effect of dispersing the external stress applied to the implant.

In addition, while being volumetrically increased, the polymeric compound placed in depth in a living body allows the implant to come into close contact with the bone, thus exceptionally augmenting the stability of the implant in the early stage of the implantation.

Further, because the polymeric compound embedded together with an implant in a living body displays superior osteoconductivity, the osseointegration of the implant into neighboring bones is further strengthened with the lapse of time.

A better understanding of the present invention may be obtained in light of following examples which are set forth to illustrate, but are not to be construed to limit, the present invention. In the following examples, prevailing Branemark implants in current use were coated with or without chitosan and implanted in living bodies. In either case, the implants were tested for their stability in the early stage of the implantation.

### EXAMPLES I THROUGH XVIII (not part of the invention)

A set of test samples were prepared by coating chitosan on the surfaces of implants. Another set of test samples were un-coated implants. The test samples each were embedded in a knee bone of a pig. 3-4 days after this implantation, each of the test samples was laterally applied with a force of 20 N and then, the displacement of the implants was measured. From this, the stiffness of the implants anchored in the bones was evaluated and is given as shown in Table I below.

**TABLE 1**

| Unit : Kg/mm² | | |
|---|---|---|
| Sample | Uncoated | Coated |
| 1 | 32.519 | 63.087 |
| 2 | 54.218 | 30.895 |
| 3 | 24.035 | 58.201 |
| 4 | 52.697 | 46.919 |
| 5 | 30.404 | 33.772 |
| 6 | 35.403 | 69.154 |
| 7 | 44.800 | 39.016 |
| 8 | 30.020 | 26.009 |
| 9 | 21.980 | 71.826 |
| 10 | 21.500 | 42.172 |
| 11 | 35.960 | 44.695 |
| 12 | 38.560 | 50.578 |
| 13 | 19.252 | 39.016 |
| 14 | 35.549 | 26.009 |
| 15 | 52.939 | 71.826 |
| 16 | 25.321 | 42.172 |
| 17 | 47.925 | 44.695 |
| 18 | 22.022 | 50.578 |
| Average | 34.728 | 47.108 |
| Std.Deviat'n | 11.668 | 14.956 |
| Shear Stress Test | | 0.011 |

In the table, higher values for the implants mean that the implants are laterally moved in shorter distances when the same force is applied. It is apparent from the table that the displacement of the implants is smaller upon the lateral application of a force of 20 N to the implants as they are of larger stiffness. Therefore, since the coated implants with chitosan have larger stiffness than do the uncoated ones in each samples, the coating with chitosan endows the implants with great stability in the early stage of the implantation. In detail, the coated implants are more improved in the initial stability than the uncoated ones by about 36%.

Besides chitosan, examples of the naturally occurring compounds which can be as a coat for a medical implant include cellulose polymers, such as cellulose, cellulose acetate, cellulose nitrate and ethyl cellulose, protein polymers, such as gelatin and collagen, guar gum, and cellulose acetate butyrate. Examples of the synthetic polymers useful as such a coat include poly-L-lactide, poly-D-L-lactide, polyglycolide-lactide, polyacrylonitrile, polyvinylacetate, polyvinylalcohol, poly γ-benzyl-L-glutamate, polyphosphazene, polyalkyleneoxalate, polydimethylsiloxane, polyurethane, polyetherurethane amide, polyesterurethane and the mixtures thereof.

As described hereinbefore, when the implant coated with a polymeric material of hydrophilicity and osteoconductivity is embedded in depth in a living body, the polymeric material absorbs moisture to swell up, bringing about an effect of dispersing the external stress applied to the implant. In addition, the implant are brought into close contact with the bone of the graft site by virtue of the volumetric increase and thus, obtains remarkably improved stability in the early stage of the implantation. Furthermore, the polymer of hydrophilicity and osteoconductivity more strengthens the osseointegration of the implant in the bone as time passes.

## Claims

1. An artificial tooth for implanting in a dental root, comprising a polymeric coating which swells in use by absorbing moisture, **characterized in that** the polymeric coating is chitosan and **in that** in use swelling of the polymeric coating disperses external stress applied to the artificial tooth.

## Patentansprüche

1. Künstlicher Zahn für das Implantieren in eine Zahnwurzel, eine Polymerbeschichtung umfassend, die beim Gebrauch durch die Aufnahme von Feuchtigkeit anschwillt, **dadurch gekennzeichnet, dass** die Polymerbeschichtung Chitosan ist und **dadurch**, dass beim Gebrauch das Anschwellen der Polymerbeschichtung die Belastung von außen verteilt, die auf den künstlichen Zahn einwirkt.

## Revendications

1. Dent artificielle à implanter dans une racine dentaire, comprenant un revêtement polymère qui gonfle à l'usage par absorption de l'humidité, **caractérisée en ce que** le revêtement polymère est en chitosan **et en ce qu**'à l'usage le gonflement du revêtement polymère disperse l'effort exteme qui s'exerce sur la dent artificielle.
